# EUROPÄISCHE PATENTSCHRIFT

(11) **EP 2 539 060 B1**
(45) Veröffentlichungstag und Bekanntmachung des Hinweises auf die Patenterteilung: **04.06.2014**
(21) Anmeldenummer: 11702915.7
(22) Anmeldetag: 21.01.2011
(51) Int. Cl.: B01F 13/00, B01F 11/00, A61C 19/00, A61F 2/46

(54) **MISCH- UND AUSTRAGSYSTEM UMFASSEND EINE SPRITZENARTIGE MISCHVORRICHTUNG MIT DISTAL BEDIENBAREM MISCHELEMENT**
MIXING AND DISPENSING SYSTEM WIITH A SYRINGE-LIKE MIXING DEVICE HAVING A DISTALLY OPERABLE MIXING ELEMENT
SYSTÈME MÉLANGEUR ET DISTRIBUTEUR COMPRENANT UN DISPOSITIF DE MÉLANGE DE TYPE SERINGUE À ÉLÉMENT DE MÉLANGE ACTIONNABLE DISTALEMENT

(30) Priorität: 22.02.2010 CH 219102010
(43) Veröffentlichungstag der Anmeldung: 02.01.2013
(73) Patentinhaber: Medmix Systems AG, 6343 Rotkreuz (CH)
(72) Erfinder: GRETER, Andy, CH-6343 Rotkreuz (CH)
(74) Vertreter: Detken, Andreas
(86) Internationale Anmeldenummer: PCT/CH2011/000006
(87) Internationale Veröffentlichungsnummer: WO 2011/100852

(56) Entgegenhaltungen:
- WO-A2-2006/023430
- US-A- 5 252 301
- US-A1- 2009 112 157

## Beschreibung

### TECHNISCHES GEBIET

Die vorliegende Erfindung betrifft eine Mischvorrichtung, die nach Art einer Spritze aufgebaut ist, mit einem Gehäuse, das eine im wesentlichen zylindrische Mischkammer begrenzt, und einem in der Mischkammer verschiebbar angeordneten Kolben, wobei ein Mischelement vom distalen Ende her in die Mischkammer eingeschoben ist.

### STAND DER TECHNIK

In verschiedenen Bereichen der Technik, darunter auch in verschiedenen medizinischen Anwendungen, ist es erforderlich, zwei oder mehr Komponenten unmittelbar vor ihrer Anwendung miteinander zu mischen. Ein wichtiges Beispiel ist die Herstellung eines Knochenzements aus einer pulverförmigen Komponente und einem flüssigen Monomer. Die beiden Komponenten werden getrennt voneinander gelagert. Erst unmittelbar vor der Anwendung werden die Komponenten gemischt, wodurch ein Aushärt- oder Geliervorgang in Gang gesetzt wird. Daher muss das durch die Mischung resultierende Produkt zeitnah nach dem Mischvorgang ausgetragen werden. Andere Beispiele sind die Herstellung eines medizinischen Klebers oder eines Medikaments aus zwei oder mehr Komponenten.

Aus dem Stand der Technik sind zu diesem Zweck Mischvorrichtungen bekannt, bei denen die zu mischenden Komponenten in eine zylindrische Mischkammer eingefüllt werden und in dieser Mischkammer durch ein Mischelement vermischt werden, und bei denen das Mischprodukt anschliessend mittels eines Kolbens durch eine distale Abgabeöffnung aus der Mischkammer ausgetragen wird. Insbesondere sind Mischvorrichtungen bekannt, die spritzenartig aufgebaut sind. In derartigen Mischvorrichtungen ist die Mischkammer in einem Gehäuse ausgebildet, das zwischen zwei Fingern einer Hand ergriffen werden kann, und der Kolben ist mit einer Kolbenstange verbunden, die an ihrem freien Ende eine Daumenauflage für den Daumen derselben Hand aufweist. Dadurch kann das Mischprodukt nach dem Mischvorgang in der selben Weise wie bei einer handelsüblichen Injektionsspritze ausgetragen werden. Derartige Mischvorrichtungen eignen sich besonders zum Mischen und Austragen verhältnismässig kleiner Mengen.

Eine derartige spritzenartige Mischvorrichtung ist z.B. in WO 2009/105905 angegeben. Bei dieser Vorrichtung wird das Mischelement von derselben (proximalen) Seite her betätigt wie der Kolben. Um das Mischelement unabhängig vom Kolben betätigen zu können, ist eine Betätigungsstange für das Mischelement durch den Kolben hindurchgeführt. An der Betätigungsstange ist eine Kolbenstange schwenkbar angelenkt. Zum Vorschieben des Kolbens wird die Kolbenstange parallel zur Betätigungsstange ausgerichtet, wobei sie die Betätigungsstange teilweise radial umgreift und am Kolben angreift. Zum Mischen des Mischgutes wird die Kolbenstange dagegen quer zur Betätigungsstange ausgerichtet. In dieser Orientierung wird die Kolbenstange durch den Benutzer ergriffen und als Handgriff zum Hin- und Herbewegen der Betätigungsstange verwendet. Eine durch den Kolben hindurch geführte Betätigungsstange ist für sehr kleine Mischkammern allerdings nicht geeignet, da der Durchmesser der Betätigungsstange im Verhältnis zum Kolbendurchmesser zu gross wird, und eine solche Vorrichtung ist in ergonomischer Hinsicht nicht optimal.

Aus dem Stand der Technik sind auch spritzenartige Mischvorrichtungen bekannt, bei denen die Betätigungsstange durch das distale, dem Kolben abgewandte Ende in die Mischkammer eingeführt ist. Eine solche Vorrichtung ist z. B. in WO 2009/048366 angegeben. Bei dieser Mischvorrichtung ist die Betätigungsstange hohl ausgebildet und dient zum Zuführen mindestens einer Komponente in die Mischkammer, in der eine andere Komponente schon vorliegt. Um das fertige Produkt auszutragen, wird die Betätigungsstange vom Mischelement getrennt, während das Mischelement in der Mischkammer verbleibt. Anschliessend wird am distalen Ende der Mischvorrichtung eine Hohlnadel zum Austragen der Mischung angebracht. An dieser Vorrichtung ist nachteilig, dass weitere Komponenten lediglich durch die hohle Betätigungsstange hindurch in die Mischkammer eingebracht werden können, die konstruktionsbedingt einen relativ kleinen Innendurchmesser hat. Dadurch eignet sich eine solche Mischvorrichtung nicht dazu, nachträglich z.B. hochviskose oder pulverförmige Komponenten in die Mischkammer einzubringen. Ausserdem ist die Handhabung der Mischvorrichtung relativ umständlich. Indem die Betätigungsstange für das Mischelement innen hohl sein muss, wird zudem die Stabilität der Betätigungsstange beeinträchtigt.

Die US 2009/0112157 offenbart ein Misch- und Austragsystem gemäß dem Oberbegriff des Anspruchs 1. Ein Kolben begrenzt die Mischkammer in Richtung des proximalen Endes. Im Gehäuse ist ein Mischelement angeordnet. Mit dem Mischelement ist eine Betätigungsstange verbunden. Diese ragt aus dem distalen offenen Ende der Mischkammer heraus. Die Bestätigungsstange ist hohl. Während des Mischvorgangs ist ein Verschlussstab in die Betätigungsstange eingeführt und verschliesst diese. Zum Austragen des Mischguts wird der Verschlussstab aus der Betätigungsstange in die distale Richtung herausgezogen, und das Mischgut wird durch die hohle Betätigungsstange hindurch ausgetragen.

Für grössere Mengen eines Mischguts sind aus dem Stand der Technik Mischvorrichtungen bekannt, bei denen das Mischprodukt mit Hilfe eines Pistolendispensers oder ähnlicher Hilfsmittel ausgetragen wird. Beispiele für derartige Vorrichtungen finden sich z.B. in US 5,842,785 oder US 2005/0128867. Aufgrund ihrer Grösse und Komplexität sind derartige Vorrichtungen jedoch nicht zum Herstellen und Austragen von kleinen Mengen eines Mischprodukts geeignet.

### DARSTELLUNG DER ERFINDUNG

Es ist daher eine Aufgabe der vorliegenden Erfindung, ein Misch- und Austragsystem zum Mischen eines Mischguts anzugeben, das einfach aufgebaut ist, das sich für die Herstellung von kleinen Mengen eines Mischprodukts eignet, bei dem das Mischprodukt nach Art einer Injektionsspritze auf einfache Weise ausgetragen werden kann, und bei dem die zu mischenden Komponenten auf einfache Weise eingefüllt werden können.

Diese Aufgabe wird durch ein Misch- und Austragsystem mit den Merkmalen des Anspruchs 1 gelöst. Weiterbildungen der Mischvorrichtung sind in den abhängigen Ansprüchen angegeben.

Es wird also ein Misch- und Austragsystem zum Mischen eines Mischguts, insbesondere von mindestens zwei Komponenten, vorgeschlagen. Das Misch- und Austragsystem umfasst eine Mischvorrichtung, aufweisend:
ein Gehäuse, das eine im wesentlichen zylindrische Mischkammer begrenzt, wobei die Mischkammer eine Längsrichtung definiert und ein distales offenes Ende sowie ein proximales offenes Ende aufweist, und wobei das Gehäuse derart dimensioniert ist, das es zwischen zwei Fingern einer ersten Hand eines Benutzers ergreifbar ist;
einen in der Mischkammer entlang der Längsrichtung verschiebbar angeordneten Kolben, der die Mischkammer in Richtung des proximalen Endes dichtend begrenzt;
eine mit dem Kolben verbundene Kolbenstange, die aus dem proximalen Ende des Gehäuses herausragt und die eine Daumenauflage aufweist, wobei die Daumenauflage derart angeordnet ist, dass sie es dem Benutzer erlaubt, die Kolbenstange mit dem Daumen der ersten Hand in die distale Richtung vorzuschieben, wenn der Benutzer das Gehäuse zwischen zwei Fingern der ersten Hand hält;
ein in der Mischkammer entlang der Längsrichtung verschiebbares Mischelement;
eine mit dem Mischelement distal verbundene Betätigungsstange, die aus dem distalen offenen Ende der Mischkammer herausragt; und
eine erste Verschlusskappe, die am distalen offenen Ende der Mischkammer lösbar mit dem Gehäuse in Eingriff steht, die das distale offene Ende der Mischkammer verschliesst, und die eine Durchgangsöffnung aufweist, durch welche die Betätigungsstange hindurchgeführt ist.

Dabei ist die Mischvorrichtung derart ausgebildet, dass die erste Verschlusskappe gemeinsam mit der Betätigungsstange und dem Mischelement vollständig vom Gehäuse entfernbar ist.

Auf diese Weise können einerseits die zu mischenden Komponenten auf einfache Weise in die Mischkammer eingefüllt werden, insbesondere auch dann, wenn es sich bei einer der Komponenten um eine hochviskose Flüssigkeit, um eine pulverförmige Substanz oder eine propfenförmige Festsubstanz handeln sollte. Die Mischkammer kann aber auch bereits unter hygienischen Bedingungen mit mindestens einer Komponente vorgefüllt sein. Andererseits kann das Mischelement auf sehr einfache Weise aus der Mischkammer entfernt werden, um das Produkt austragen zu können, ohne dass der Austragvorgang durch die Anwesenheit des Mischelements und der Betätigungsstange behindert wird. Gleichzeitig bietet eine solche Vorrichtung die Möglichkeit, die Betätigungsstange mit einem Handgriff zu versehen, der eine einfache und ergonomische Handhabung beim Mischen sicherstellt. Die Betätigungsstange kann ohne Weiteres derart dimensioniert werden, dass die nötige Stabilität der Betätigungsstange jederzeit gewährleistet ist. Indem dabei die Mischvorrichtung ansonsten nach Art einer normalen Injektionsspritze ausgestaltet ist, bietet die Mischvorrichtung zudem eine extrem einfache Handhabung zum Austragen des Mischprodukts. Insgesamt ist eine sterile Handhabung der Komponenten beim Befüllen, Mischen und Austragen gewährleistet.

Um eine sichere Führung der Betätigungsstange zu gewährleisten, kann die erste Verschlusskappe ein Führungselement aufweisen, das eine Länge aufweist, die mindestens einem Drittel des Innendurchmessers der kreiszylindrischen Mischkammer entspricht, und das sich bevorzugt in die Mischkammer hinein erstreckt. Um eine gute Stabilität gegen Verkippen zu erreichen, liegt das Führungselement vorzugsweise an mindestens drei Stellen, bevorzugt umlaufend, innen an einer Begrenzungswand der Mischkammer an. Im Führungselement (das auch als Führungsbuchse oder Führungshülse bezeichnet werden kann) ist die Betätigungsstange geführt. Dazu weist das Führungselement eine Durchgangsöffnung, insbesondere eine durchgehende Bohrung auf, durch welche die Betätigungsstange hindurchgeführt ist. Die Durchgangsöffnung dient bevorzugt unmittelbar zu einer gleitenden Führung der Betätigungsstange und ist dazu genügend lang ausgebildet, um ein Verkippen der Betätigungsstange gegenüber dem Führungselement zu verhindern. Insgesamt wird auf diese Weise eine besonders hohe Stabilität der Führung der Betätigungsstange gewährleistet.

Vorzugsweise ist an der ersten Verschlusskappe eine Abstreiflippe ausgebildet, die umlaufend an der Betätigungsstange anliegt, um ein Herausdringen des Mischguts während des Mischvorgangs zu verhindern. Die Abstreiflippe ist bevorzugt am Führungselement, insbesondere in einem proximalen Endbereich des Führungselements ausgebildet.

In einem proximalen Endbereich der ersten Verschlusskappe, insbesondere des Führungselements, kann eine Vertiefung ausgebildet sein, die sich in distaler Richtung verjüngt. Dies ist insbesondere dann vorteilhaft, wenn das Mischelement sich ebenfalls in der distalen Richtung verjüngt. In diesem Fall nimmt die Vertiefung das distale Ende des Mischelements auf, wenn sich das Mischelement in einer distalen Endstellung befindet. Auf diese Weise ist sichergestellt, dass die in der Mischkammer aufgenommenen Komponenten auch in einem distalen Bereich der Mischkammer, der unmittelbar an die Verschlusskappe angrenzt, effizient durchmischt werden. Falls eine Abstreiflippe vorhanden ist, ist diese bevorzugt in einem distal an die Vertiefung anschliessenden Bereich des Führungselements ausgebildet.

Entsprechend kann auch die Form eines distalen Endbereichs des Kolbens komplementär zum Mischelement ausgestaltet werden und insbesondere einen sich in distaler Richtung verjüngenden Endbereich aufweisen, wenn das Mischelement eine sich distal verjüngende Aufnahme für den Endbereich des Kolbens bildet. Auf diese Weise ist auch eine effiziente Durchmischung des Mischguts in einem unmittelbar an den Kolben angrenzenden proximalen Endbereich der Mischkammer gewährleistet.

In einer möglichen konkreten Ausgestaltung weist das Mischelement mindestens einen, bevorzugt mindestens drei in die proximale Richtung geneigte, sich von der zentralen Mittelachse der Mischkammer radial nach aussen erstreckende Arme auf. Die Arme können gedanklich als Teil einer zur distalen Richtung hin zulaufenden Rotationsfläche, insbesondere einer Kegelfläche aufgefasst werden. Im Bereich der zentralen Mittelachse der Mischkammer sind die Arme mit der Betätigungsstange verbunden. An ihren freien Enden können die Arme aufgeweitet sein und/oder weitere Strukturen aufweisen, beispielsweise können die Arme an ihren freien Enden T-förming ausgebildet sein. Unabhängig von der konkreten Ausgestaltung des Mischelements liegt dieses bevorzugt mindestens bereichsweise an der Begrenzungswand der Mischkammer an, um eine effiziente Durchmischung des Mischguts auch im Bereich der Begrenzungswand sicher zu stellen.

Das Mischelement ist bevorzugt nicht nur entlang der Längsrichtung innerhalb der Mischkammer hin- und her bewegbar, sondern auch um die Längsrichtung drehbar. Es wird dazu bevorzugt manuell bewegt. Hierzu weist die Mischvorrichtung vorzugsweise einen Handgriff auf, der mit der Betätigungsstange an deren dem Mischelement abgewandten, distalen Ende verbunden ist. Dieser Handgriff ist dazu ausgebildet, die Betätigungsstange mit einer zweiten Hand des Benutzers manuell entlang der Längsrichtung hin und her zu bewegen und um die Längsrichtung zu drehen, während der Benutzer das Gehäuse mit der ersten Hand hält. Der Handgriff kann speziell an die Anatomie der menschlichen Hand angepasst sein, um eine gute Ergonomie zu gewährleisten. So kann sich der Handgriff zum Beispiel keilförmig in die distale Richtung aufweiten und eine asymmetrische Form bezüglich einer Rotation um die Längsrichtung aufweisen, insbesondere senkrecht zur Längsrichtung abgeplattet sein.

Da die Betätigungsstange gemeinsam mit der Verschlusskappe und dem Mischelement vollständig vom Gehäuse entfernbar ist, ist es nicht nötig, dass die Betätigungsstange vom Mischelement lösbar ist. Um die Mischvorrichtung besonders einfach aufbauen und herstellen zu können, ist es insbesondere bevorzugt, wenn die Betätigungsstange unlösbar mit dem Mischelement verbunden ist, insbesondere einstückig mit dem Mischelement gefertigt ist.

Um die Verschlusskappe auf einfache Weise am Gehäuse zu fixieren, kann am Gehäuse im Bereich des distalen Endes der Mischkammer ein Aussengewinde ausgebildet sein. Die Verschlusskappe weist dann einen Mantelbereich mit einem Innengewinde auf, das mit dem Aussengewinde in einem Gewindeeingriff steht, wobei der Mantelbereich bevorzugt das distale Ende des Gehäuses umgreift. Alternativ sind auch andere Lösungen denkbar. So kann insbesondere am Gehäuse im Bereich des distalen Endes der Mischkammer auch eine Bajonettführung in Form einer Bajonettnut ausgebildet sein, und die Verschlusskappe kann einen Bajonettzapfen aufweisen, der mit der Bajonettführung im Eingriff steht. Umgekehrt kann auch ein Bajonettzapfen am Gehäuse und eine Bajonettführung in Form einer Nut im Mantelbereich der Verschlusskappe ausgebildet sein.

Um das Mischelement selbst dann einfach aus der Mischkammer entnehmen zu können, wenn dieses von innen an der Begrenzungswand der Mischkammer anliegt, ist das distale Ende der Mischkammer bevorzugt derart ausgebildet, dass es sich in die distale Richtung gegenüber dem Rest der Mischkammer nicht verjüngt. In anderen Worten bildet das distale Ende der Mischkammer bevorzugt eine Zugangsöffnung zur Mischkammer, deren Durchmesser mindestens ebenso gross ist wie der Durchmesser der Mischkammer in einem proximal beabstandet zum distalen Ende gelegenen zylindrischen Bereich, in dem das Mischelement verschiebbar ist.

Die vorstehend beschriebene Mischvorrichtung kann vorteilhaft mit weiteren Teilen zu einem Misch- und Austragsystem kombiniert werden erfindungsgemäß wird die Mischvorrichtung mit einer zweiten Verschlusskappe kombiniert, die nach Entfernung der ersten Verschlusskappe lösbar im Bereich des distalen offenen Endes der Mischkammer mit dem Gehäuse in Eingriff bringbar ist. Dabei verschliesst die zweite Verschlusskappe das distale offene Ende der Mischkammer im Wesentlichen und weist eine Austragöffnung auf, durch die hindurch ein in der Mischkammer vorliegendes Mischprodukt durch Druck auf die Kolbenstange in distale Richtung austragbar ist.

Zum Austragen des Mischprodukts wird also in der Regel die erste Verschlusskappe gemeinsam mit dem Mischelement und der Betätigungsstange vom Gehäuse abgenommen und die zweite Verschlusskappe aufgesetzt. Durch die in der zweiten Verschlusskappe ausgebildete Austragöffnung kann das Mischprodukt nun ausgetragen werden.

Die zweite Verschlusskappe kann insbesondere dazu ausgebildet sein, dass Zubehörteile an diese Kappe anschliessbar sind. Dazu kann die zweite Verschlusskappe einen die Austragöffnung umgebenden Anschlussbereich für ein solches Zubehörteil aufweisen. Dieser Anschlussbereich weist bevorzugt eine in distale Richtung konisch zulaufende Mantelfläche auf, um das Zubehörteil über eine übliche Kegelflächenverbindung anzuschliessen. Insbesondere ist der Anschlussbereich bevorzugt als männlicher Luer-Kegel ausgebildet und weist dazu eine Neigung von ca. 6% gegenüber der Längsrichtung auf, gemäss den Normen DIN EN 1707:1996 und DIN EN 20594-1:1993.

Um das Zubehörteil am Anschlussbereich zu sichern, kann die zweite Verschlusskappe eine den Anschlussbereich radial umgebende, beabstandet zum Anschlussbereich drehfest oder drehbar angeordnete Sicherungshülse oder Überwurfmutter mit einem zum Anschlussbereich hinweisenden Innengewinde aufweisen. Der Anschlussbereich und die Sicherungshülse können gemeinsam insbesondere als Luer-Lock gemäss den oben genannten Normen ausgebildet sein.

Das Misch- und Austragssystem kann zudem ein Verschlussteil aufweisen, das am Anschlussbereich der zweiten Verschlusskappe lösbar befestigbar ist, um die Austragsöffnung zu verschliessen. Dieses Verschlussteil kann insbesondere eine Hülse mit einem weiblichen Luer-Kegel aufweisen, die mit einem zum Innengewinde der Sicherungshülse komplementären Aussengewinde versehen sein kann.

Das Misch- und Austragssystem kann zudem eines oder mehrere Zubehörteile umfassen. Die Zubehörteile können lösbar mit der Austragöffnung der zweiten Verschlusskappe verbindbar sein, z.B. auf den Anschlussbereich aufsteckbar sein, oder sie können unlösbar mit der Austragöffnung verbunden sein und mit der zweiten Verschlusskappe gemeinsam eine Einheit bilden. Zubehör kann auch direkt in die erste Verschlusskappe integriert sein. Als Zubehörteile kommen insbesondere in Frage: eine Austragdüse, eine Sprüheinrichtung, eine Hohlnadel oder ein Mischrohr, in dem ein weiteres Mischelement, z.B. ein statischer Mischwendel oder ein dynamisches Mischelement vorgesehen sein kann. Die vorstehende Aufzählung ist selbstverständlich nicht abschliessend. Zudem ist es möglich, nach dem Mischvorgang die Mischeinheit zu entfernen und das Gemisch direkt und ohne aufgesetztes Zubehör auszustossen.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1.: eine Seitenansicht einer erfindungsgemässen Mischvorrichtung;
- Fig. 2: eine perspektivische Schnittdarstellung der Mischvorrichtung der Fig. 1;
- Fig. 3: einen vergrösserten Ausschnitt aus der Fig. 2;
- Fig. 4: eine perspektivische Explosionsdarstellung der Mischvorrichtung der Fig. 1;
- Fig. 5: eine Seitenansicht des Gehäuses und der Kolbeneinheit der Fig. 1 ohne Mischeinheit;
- Fig. 6: eine Seitenansicht des Gehäuses und der Kolbeneinheit mit einer zweiten Verschlusskappe;
- Fig. 7: eine Seitenansicht der Vorrichtung der Fig. 6 mit einem zusätzlichen Verschlussteil;
- Fig. 8: einen zentralen Längsschnitt durch die Vorrichtung der Fig. 7;
- Fig. 9: eine Seitenansicht der Vorrichtung der Fig. 6 mit angeschlossener Hohlnadel;
- Fig. 10: eine Seitenansicht der Vorrichtung der Fig. 6 mit angeschlossenem Mischrohr;
- Fig. 11: einen zentralen Längsschnitt durch die Vorrichtung der Fig. 10; sowie
- Fig. 12: einen zentralen Längsschnitt durch eine Vorrichtung mit Gehäuse, Kolben und einem integral mit einer Verschlusskappe ausgebildeten Mischrohr.

### BESCHREIBUNG BEVORZUGTER AUSFÜHRUNGSFORMEN

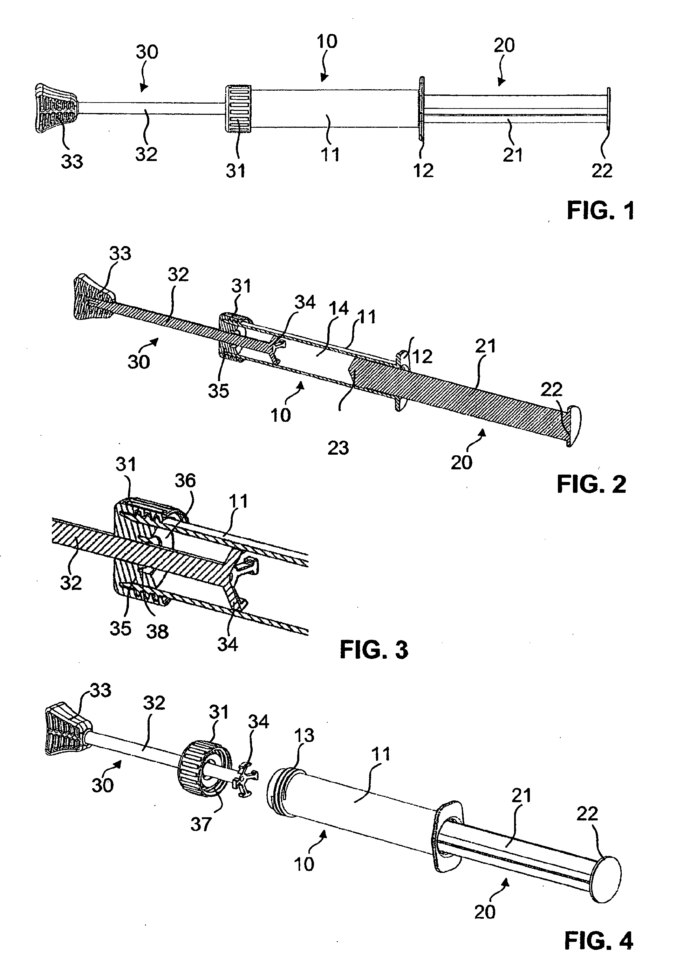

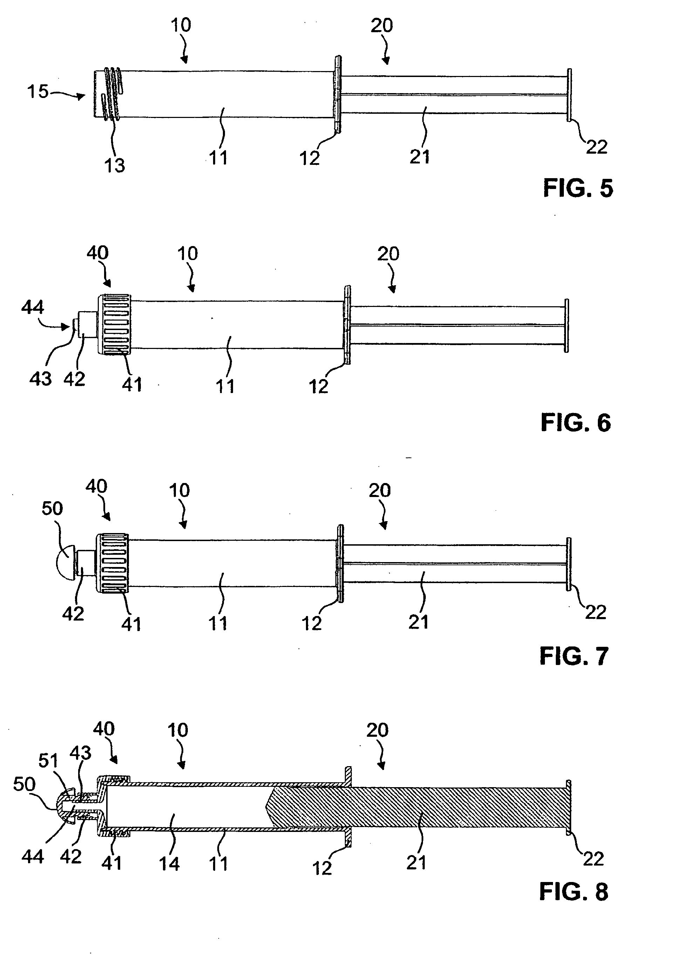

In den Figuren 1 bis 4 ist ein bevorzugtes Ausführungsbeispiel einer erfindungsgemässen Mischvorrichtung illustriert. Die Mischvorrichtung umfasst ein Gehäuse 10, dass mit seiner Seitenwand 11 eine zylindrische Mischkammer 14 begrenzt. Die Mischkammer 14 definiert mit ihrer zentralen Mittelachse eine Längsrichtung und weist ein distales offenes Ende 15 (Fig. 5) sowie ein proximales offenes Ende auf.

Durch das proximale offene Ende ist eine Kolbeneinheit 20 in die Mischkammer 14 eingeschoben. Die Kolbeneinheit umfasst einen Kolben 23, der umlaufend dichtend an der Seitenwand 11 (d.h. der Begrenzungswand der Mischkammer 14) anliegt, eine einstückig damit verbundene Kolbenstange 21 mit kreuzförmigen Querschnitt, sowie eine am proximalen Ende der Kolbenstange 21 ausgebildete Daumenauflage 22. Das Gehäuse 10 und die Kolbeneinheit 20 sind in der Fig. 5 alleine dargestellt.

Das offene distale Ende 15 der Mischkammer 14 ist durch eine erste Verschlusskappe 31 verschlossen, die auf ein Aussengewinde 13 aufgeschraubt ist. Die Verschlusskappe 31 weist ein hülsen- oder buchsenartiges Führungselement 35 auf, das sich axial in proximaler Richtung in die Mischkammer 14 hinein erstreckt und mit seiner äusseren Umfangsfläche innen an der Seitenwand 11 des Gehäuses 10 anliegt. Das Führungselement 35 weist eine zentrale Bohrung entlang der Längsrichtung auf, in der eine Betätigungsstange 32 geführt ist.

Am proximalen Ende der Betätigungsstange 32 ist ein Mischelement 34 einstückig mit der Betätigungsstange ausgebildet. Das Mischelement 34 umfasst vier sich von der zentralen Mittelachse aus radial nach aussen erstreckende Arme, die zur Längsrichtung um ca. 60 bis 75 Grad gewinkelt verlaufen und sich zur proximalen Richtung hin neigen. An ihren freien Enden sind die Arme T-förmig verdickt. Mit diesen freien Enden liegen die Arme innen an der Seitenwand 11 des Gehäuses 10 an.

In einem proximalen Endbereich des Führungselements 35 ist eine flache, konische Vertiefung 36 ausgebildet, an die sich distal eine zylindrische, sacklochartige Vertiefung kleineren Durchmessers anschliesst. In der zylindrischen Vertiefung ist wiederum eine umlaufende Abstreiflippe 38 ausgebildet. Die Abstreiflippe 38 liegt dichtend an der Betätigungsstange an. Sie ragt innerhalb der zylindrischen Vertiefung, ausgehend von der distalen Stirnseite der Vertiefung, im Wesentlichen in die proximale Richtung, wobei sich die Dicke der Abstreiflippe in proximaler Richtung verjüngt.

Die Form der flachen Vertiefung 36 ist derart komplementär zur Form des Mischelements 34, dass das Mischelement 34 teilweise in der Vertiefung 36 aufgenommen ist, wenn es mittels der Betätigungsstange 32 ganz in eine distale Endstellung zurückgezogen wird. Umgekehrt ist der distale Endbereich des Kolbens 23 ebenfalls derart komplementär zur Form des Mischelements 34 ausgebildet, dass die Arme des Mischelements 34 auf dem distalen Endbereich des Kolbens 23 aufliegen, wenn das Mischelement 34 mittels der Betätigungsstange 32 in eine proximale Endstellung geschoben wird, in der es am Kolben 23 anliegt. Anders ausgedrückt bildet das Mischelement in dieser Stellung eine Aufnahme für das in distaler Richtung zulaufende Ende des Kolbens 23.

Am distalen Ende der Betätigungsstange 32 ist ein Handgriff 33 angebracht, insbesondere aufgeklickt oder aufgepresst. Der Handgriff 33 weist eine abgeflachte Form auf und weitet sich zur distalen Seite hin keilförmig auf. Dadurch bildet er an seinem distalen Ende eine relativ grosse Auflagefläche für den Handballen einer Hand des Benutzers.

Das Mischelement 34, die Betätigungsstange 32, der Handgriff 33 und die erste Verschlusskappe 31 bilden gemeinsam eine Mischeinheit 30. Diese wird bei der Herstellung montiert, indem zunächst die Betätigungsstange 32 mit dem einstückig daran angebrachten Mischelement 34 von der proximalen Seite her durch die Durchgangsbohrung des Führungselements 35 hindurch gesteckt wird und anschliessend der Handgriff 33 auf das distale Ende der Betätigungsstange 32 aufgeklickt wird. Die gesamte Mischeinheit 30 ist als Ganze vom Gehäuse 10 entfernbar, wie dies in der Fig. 4 angedeutet ist.

Um mit dieser Mischvorrichtung eine Mischung aus mehreren Komponenten herzustellen, werden zunächst die Komponenten bei zurückgezogener Kolbeneinheit 20 und abgenommener Mischeinheit 30 in die Mischkammer 14 eingefüllt. Anschliessend wird die Mischeinheit 30 auf das offene Ende 15 der Mischkammer 14 aufgesetzt, und die erste Verschlusskappe 31 wird auf das Gewinde 14 aufgeschraubt. Um die Komponenten zu durchmischen, wird nun das Mischelement 34 in der Mischkammer 14 entlang der Längsrichtung hin- und herbewegt und um die Längsrichtung verdreht. Dazu ergreift der Benutzer das Gehäuse 10 mit seiner ersten Hand, während er den Handgriff 33 in seine zweite Hand nimmt. Wenn das Mischgut genügend durchmischt ist, zieht der Benutzer die Betätigungsstange 32 in die distale Richtung zurück, bis das Mischelement 34 am Führungselement 35 der Verschlusskappe 31 anschlägt. Anschliessend schraubt der Benutzer die Verschlusskappe 31 vom Gehäuse 10 ab und entfernt die vollständige Mischeinheit 30.

Um das Produkt nun aus der Mischkammer 14 auszutragen, setzt der Benutzer eine zweite Verschlusskappe 40 auf das distale offene Ende der Mischkammer 14 auf. Dies ist in der Fig. 6 illustriert. Die zweite Verschlusskappe 40 weist wie die erste Verschlusskappe 31 einen Mantelbereich 41 auf, an dem ein Innengewinde ausgebildet ist, das in das Aussengewinde 13 am Gehäuse 10 eingreift. Die Verschlusskappe 40 weist eine Austragöffnung 44 auf, die sich entlang der zentralen Mittelachse der Mischkammer 14 in der Längsrichtung erstreckt und die von einem Anschlussbereich 43 in Form eines männlichen Luer-Kegels umgeben ist. Eine fest mit der Verschlusskappe 40 verbundene Sicherungshülse 42 umgibt den Anschlussbereich 43 radial. An der Innenseite der Sicherungshülse 42 ist ein Innengewinde ausgebildet. Der Anschlussbereich 43 und die Sicherungshülse 42 bilden gemeinsam einen männlichen Luer-Lock-Anschluss für ein Zubehörteil.

Wenn die Austragöffnung 44 verschlossen werden soll, kann hierfür ein Verschlussteil 50 verwendet werden, wie es in den Figuren 7 und 8 illustriert ist. Das Verschlussteil 50 weist eine Hülse 51 auf, deren innere Umfangsfläche komplementär zum Luer-Kegel des Anschlussbereichs 43 ausgebildet ist. An seiner äusseren Umfangsfläche weist die Hülse 51 ein Aussengewinde auf, das in das Innengewinde der Sicherungshülse 42 eingeschraubt werden kann. In die distale Richtung ist die Hülse 51 durch einen pilzförmigen oder halbkugelförmigen Abschluss verschlossen.

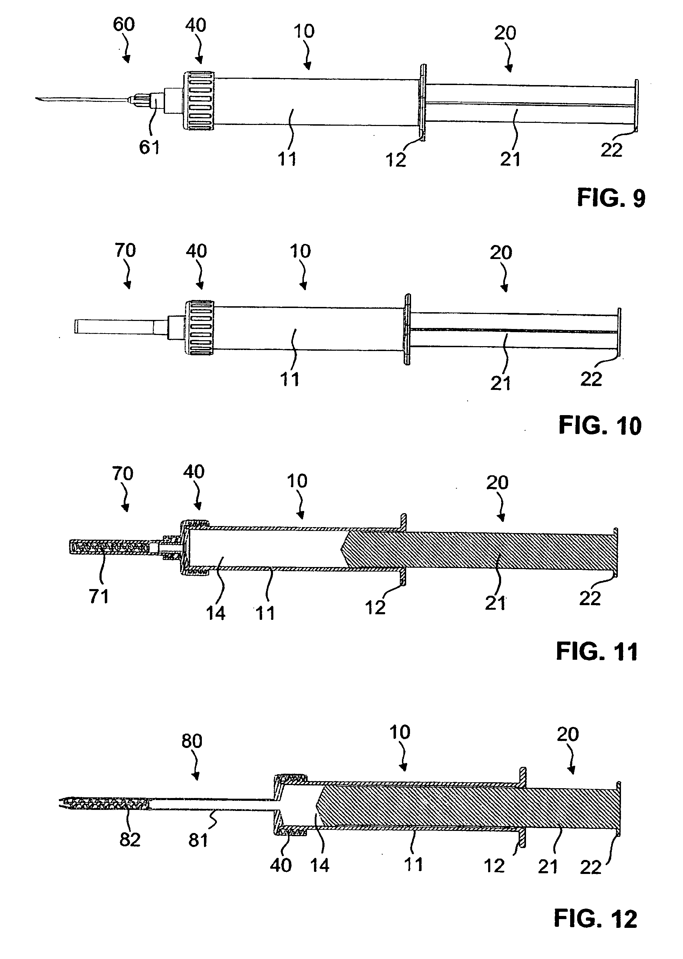

Um das Produkt aus der Mischkammer 14 auszutragen, können verschiedene Zubehörteile auf den Anschlussbereich 43 aufgesteckt und mit der Sicherungshülse 42 am Anschlussbereich gesichert werden. Dies ist in den Figuren 8-12 illustriert. Bei dem Zubehörteil kann es sich zum Beispiel um eine handelsübliche Hohlnadel 60 (Fig. 9) mit einem Anschlussbereich 61 handeln, der zum Anschlussbereich 43 der zweiten Verschlusskappe komplementär ist. Alternativ kann es sich bei dem Zubehörteil um ein Mischrohr 70 handeln (Figuren 10 und 11). Im Mischrohr 70 kann insbesondere ein weiteres Mischelement 71, z.B. in Form eines statischen Mischwendels, vorhanden sein, um eine letzte abschliessende Durchmischung des Mischguts während des Austragens zu erreichen. Eine Vielzahl weiterer Zubehörteile sind denkbar.

Die Mischvorrichtung bildet gemeinsam mit der zweiten Verschlusskappe, mit einem oder mehreren solchen Zubehörteilen und ggfs. mit dem Verschlussteil ein komplettes Misch- und Austragsystem, das komplett als Set im Handel angeboten werden kann.

Das Zubehörteil kann auch einstückig mit der zweiten Verschlusskappe 40 ausgebildet sein, wie dies in der Fig. 12 für ein Zubehörteil 80 illustriert ist. Hier ist mit der zweiten Verschlusskappe 40 einstückig ein Mischrohr 81 verbunden, in dem in einem distalen Endbereich ein Mischwendel 82 angeordnet ist. Eine Vielzahl weiterer Zubehörteile sind auch hier denkbar.

Um das Mischgut aus der Mischkammer 14 auszutragen, ergreift der Benutzer das Gehäuse 10 zwischen zwei Fingern, insbesondere dem Zeigefinger und dem Mittelfinger, einer Hand. Mit dem Daumen derselben Hand übt der Benutzer eine in distaler Richtung wirkende Kraft auf die Daumenauflage 22 aus. Um die dabei wirkende Gegenkraft besser mit den Fingern aufnehmen zu können, ohne abzurutschen, ist am proximalen Ende des Gehäuses 10 ein an sich bekannter Rückhalteflansch 12 ausgebildet. Das Austragen der Mischung aus der Mischvorrichtung erfolgt also im Wesentlichen auf dieselbe Weise, wie eine Substanz aus einer handelsüblichen Injektionsspritze verabreicht wird.

Die vorstehend dargestellte Mischvorrichtung und das darauf basierende Misch- und Austragsystem eignen sich besonders zur Herstellung relativ kleiner Mengen eines Mischguts. Insbesondere weist die Mischkammer 14 bevorzugt ein Volumen zwischen ca. 1 und 100 Millilitern auf. Die Mischvorrichtung eignet sich dabei insbesondere zum Einsatz im medizinischen Bereich, insbesondere zur Herstellung von kleinen Mengen eines Knochenzements, einer Dentalmasse, eines oral zu verabreichenden Medikaments usw. Aufgrund seiner einfachen Konstruktion und der geringen Fertigungskosten eignet sich das System zur einmaligen Verwendung als Wegwerfartikel.

Selbstverständlich sind eine Vielzahl von Abwandlungen der erfindungsgemässen Mischvorrichtung und des Misch- und Austragsystems möglich. So kann z.B. das Mischelement auch getrennt von der Betätigungsstange gefertigt werden, insbesondere auch aus einem anderen Material. Selbstverständlich sind andere Formen des Gehäuses 10, der Kolbeneinheit 20 und der Mischereinheit 30, insbesondere des Handgriffs 33, möglich. Die Verbindung zwischen den Verschlusskappen und dem Gehäuse kann auf andere als die hier dargestellte Weise erfolgen, z.B. über eine bajonettartige Verbindung. Eine Vielzahl weiterer Abwandlungen sind möglich, und die Erfindung ist keineswegs auf die vorstehenden Beispiele beschränkt.

### BEZUGSZEICHENLISTE

| | | | |
|---|---|---|---|
| 10 | Gehäuse | 37 | Innengewinde |
| 11 | Seitenwand | 38 | Abstreiflippe |
| 12 | Rückhalteflansch | 40 | zweite Verschlusskappe |
| 13 | Aussengewinde | 41 | Mantelbereich |
| 14 | Mischkammer | 42 | Sicherungshülse |
| 20 | Kolbeneinheit | 43 | Anschlussbereich |
| 21 | Kolbenstange | 44 | Austragöffnung |
| 22 | Daumenauflage | 50 | Verschlussteil |
| 23 | Kolben | 51 | Hülse |
| 30 | Mischeinheit | 60 | Hohlnadel |
| 31 | erste Verschlusskappe | 61 | Anschlussbereich |
| 32 | Betätigungsstange | 70 | Mischrohr |
| 33 | Handgriff | 71 | Mischwendel |
| 34 | Mischelement | 80 | Zubehörteil |
| 35 | Führungselement | 81 | Mischrohr |
| 36 | Vertiefung | 82 | Mischwendel |

## Patentansprüche

1. Misch- und Austragsystem, das eine Mischvorrichtung zum Mischen eines Mischguts umfasst, wobei die Mischvorrichtung aufweist:
ein Gehäuse (10), das eine im Wesentlichen zylindrische Mischkammer (14) begrenzt, wobei die Mischkammer (14) eine Längsrichtung definiert und ein distales offenes Ende (15) sowie ein proximales offenes Ende aufweist, und wobei das Gehäuse derart dimensioniert ist, dass es zwischen zwei Fingern einer ersten Hand eines Benutzers ergreifbar ist;
einen in der Mischkammer (14) entlang der Längsrichtung verschiebbar angeordneten Kolben (23), der die Mischkammer in Richtung des proximalen Endes dichtend begrenzt;
eine mit dem Kolben (23) verbundene Kolbenstange (21), die aus dem proximalen Ende der Mischkammer (14) herausragt und die eine Daumenauflage (22) aufweist, wobei die Daumenauflage (22) derart angeordnet ist, dass sie es dem Benutzer erlaubt, die Kolbenstange (21) mit dem Daumen der ersten Hand in die distale Richtung vorzuschieben, wenn der Benutzer das Gehäuse (10) zwischen zwei Fingern der ersten Hand hält;
ein in der Mischkammer (14) entlang der Längsrichtung verschiebbares Mischelement (34);
eine mit dem Mischelement (34) distal verbundene Betätigungsstange (32), die aus dem distalen offenen Ende der Mischkammer (14) herausragt; und
eine erste Verschlusskappe (31), die am distalen offenen Ende (15) der Mischkammer (14) lösbar mit dem Gehäuse (10) in Eingriff steht, die das distale offene Ende (15) der Mischkammer (14) verschliesst, und die eine Durchgangsöffnung aufweist, durch welche die Betätigungsstange (32) hindurch geführt ist, wobei die erste Verschlusskappe (31) gemeinsam mit der Betätigungsstange (32) und dem Mischelement (34) vollständig vom Gehäuse (10) entfernbar ist;
**dadurch gekennzeichnet, dass** das Misch- und Austragsystem ausserdem eine zweite Verschlusskappe (40) umfasst, die nach Entfernung der ersten Verschlusskappe (31) lösbar mit dem Gehäuse (10) im Bereich des distalen offenen Endes (15) der Mischkammer (14) in Eingriff bringbar ist, um das distale offene Ende (15) der Mischkammer (14) zu verschliessen, und die eine Austragöffnung (44) aufweist, durch die hindurch ein in der Mischkammer (14) aufgenommenes Mischprodukt durch Druck auf die Kolbenstange (21) in distaler Richtung austragbar ist.

2. Misch- und Austragsystem nach Anspruch 1, wobei die erste Verschlusskappe (31) ein Führungselement (35) aufweist, das sich in die Mischkammer (14) hinein erstreckt, das an mindestens drei Stellen, bevorzugt umlaufend, innen an einer Begrenzungswand (11) der Mischkammer (10) anliegt, und in dem die Betätigungsstange (32) geführt ist.

3. Misch- und Austragsystem nach Anspruch 1 oder 2, wobei in einem proximalen Endbereich der ersten Verschlusskappe (31) eine sich in distaler Richtung verjüngende Vertiefung (36) ausgebildet ist, um ein distales Ende des Mischelements (34) aufzunehmen, wenn sich das Mischelement (34) in einer distalen Endstellung befindet.

4. Misch- und Austragsystem nach einem der vorhergehenden Ansprüche, wobei der Kolben (23) einen sich in distaler Richtung verjüngenden Endbereich aufweist, und wobei das Mischelement (34) eine sich distal verjüngende Aufnahme für den Endbereich des Kolbens (23) bildet.

5. Misch- und Austragsystem nach einem der vorhergehenden Ansprüche, wobei die erste Verschlusskappe eine umlaufende Abstreiflippe aufweist, die an der Betätigungsstange anliegt.

6. Misch- und Austragsystem nach einem der vorhergehenden Ansprüche, wobei die Mischvorrichtung einen Handgriff (33) aufweist, der mit der Betätigungsstange (32) an ihrem dem Mischelement (34) abgewandten distalen Ende verbunden ist, und wobei der Handgriff (33) dazu ausgebildet ist, die Betätigungsstange (32) mit einer zweiten Hand des Benutzers manuell entlang der Längsrichtung hin und her zu bewegen und zu drehen, während der Benutzer das Gehäuse (10) mit der ersten Hand hält.

7. Misch- und Austragsystem nach Anspruch 6, wobei der Handgriff (33) sich keilförmig in die distale Richtung aufweitend und asymmetrisch bezüglich einer Rotation um die Längsrichtung ausgebildet ist.

8. Misch- und Austragsystem nach einem der vorhergehenden Ansprüche, wobei die Betätigungsstange (32) unlösbar mit dem Mischelement (34) verbunden ist.

9. Misch- und Austragsystem nach einem der vorhergehenden Ansprüche, wobei am Gehäuse (10) im Bereich des distalen Endes (15) der Mischkammer (14) ein Aussengewinde (13) ausgebildet ist, und wobei die erste Verschlusskappe (31) einen Mantelbereich mit einem Innengewinde (37) aufweist, das mit dem Aussengewinde (13) in Eingriff steht.

10. Misch- und Austragsystem nach einem der vorhergehenden Ansprüche, wobei das offene distale Ende (15) der Mischkammer (14) eine Zugangsöffnung zur Mischkammer (14) bildet, deren Durchmesser mindestens ebenso gross ist wie der Durchmesser der Mischkammer (14) in einem proximal beabstandet vom distalen Ende (15) gelegenen zylindrischen Bereich, in dem der Kolben (23) verschiebbar ist.

11. Misch- und Austragsystem nach einem der vorhergehenden Ansprüche, wobei die zweite Verschlusskappe (40) einen die Austragöffnung (44) umgebenden Anschlussbereich (43) für ein Zubehörteil aufweist, der eine in distaler Richtung konisch zulaufende Mantelfläche aufweist und vorzugsweise als männlicher Luerkegel ausgebildet ist.

12. Misch- und Austragsystem nach Anspruch 11, wobei die zweite Verschlusskappe (40) eine den Anschlussbereich (43) radial umgebende, drehfest oder drehbar angeordnete Sicherungshülse (42) mit einem zum Anschlussbereich (43) hin weisenden Innengewinde aufweist, um das Zubehörteil am Anschlussbereich zu sichern.

13. Misch- und Austragsystem nach Anspruch 11 oder 12, welche ausserdem einen am Anschlussbereich (43) lösbar befestigbares Verschlussteil (50) aufweist, um die Austragöffnung (44) zu verschliessen.

14. Misch- und Austragsystem nach einem der vorhergehenden Ansprüche, welches ausserdem mindestens ein Zubehörteil aufweist, welches lösbar mit der Austragöffnung (44) verbindbar ist oder unlösbar mit der Austragöffnung (44) verbunden ist und aus der folgenden Gruppe von Zubehörteilen ausgewählt ist:
eine Austragdüse;
eine Sprüheinrichtung;
eine Hohlnadel (60);
ein Mischrohr (70; 80).

## Claims

1. A mixing and discharge system comprising a mixing device for mixing a product to be mixed, the mixing device comprising:
a housing (10) that delimits an essentially cylindrical mixing chamber (14), wherein the mixing chamber (14) defines a longitudinal direction and comprises a distal open end (15) and a proximal open end, and wherein the housing is dimensioned in such a manner that it can be held between two fingers of a first hand of a user;
a piston (23) arranged in the mixing chamber (14) so as to be slidable along the longitudinal direction, which piston (23) sealingly delimits the mixing chamber in the direction of the proximal end;
a piston rod (21), connected to the piston (23), which piston rod (21) protrudes from the proximal end of the mixing chamber (14) and which piston rod comprises a thumb rest (22), wherein the thumb rest (22) is arranged in such a manner that it makes it possible for the user to advance the piston rod (21) with the thumb of the first hand in the distal direction when the user holds the housing (10) between two fingers of the first hand;
a mixing element (34) that is displaceable in the mixing chamber (14) along the longitudinal direction;
an operating rod (32) that is distally connected to the mixing element (34), which operating rod (32) protrudes from the distal open end of the mixing chamber (14); and
a first closure cap (31) that at the distal open end (15) of the mixing chamber (14) detachably engages the housing (10), which closure cap (31) closes the distal open end (15) of the mixing chamber (14) and comprises a passage opening through which the operating rod (32) is guided,
wherein the first closure cap (31) together with the operating rod (32) and the mixing element (34) is completely removable from the housing (10),
**characterised in that** the mixing and discharge system further comprises a second closure cap (40), which is configured to detachably engage the housing (10) in the region of the distal open end (15) of the mixing chamber (14) after removal of the first closure cap (31), in order to close the distal open end (15) of the mixing chamber (14), and which has a discharge opening (44) through which a mixed product present in the mixing chamber (14) can be discharged with the application of pressure on the piston rod (21) in the distal direction.

2. The mixing and discharge system according to claim 1, wherein the first closure cap (31) comprises a guide element (35) that extends into the mixing chamber (14), which guide element (35) rests against at least three positions, preferably circumferentially, on the inside against a defining wall (11) of the mixing chamber (10), with the operating rod (32) being guided in said guide element (35).

3. The mixing and discharge system according to claim 1 or 2, wherein a proximal end region of the first closure cap (31) comprises an indentation (36) that tapers off in the distal direction in order to receive a distal end of the mixing element (34) when the mixing element (34) is in a distal end position.

4. The mixing and discharge system according to any one of the preceding claims, wherein the piston (23) comprises an end region that tapers off in the distal direction, and wherein the mixing element (34) forms a distally-tapering receiving device for the end region of the piston (23).

5. The mixing and discharge system according to any one of the preceding claims, wherein the first closure cap comprises a circumferential wiping lip that rests against the operating rod.

6. The mixing and discharge system according to any one of the preceding claims, wherein the mixing device comprises a hand grip (33) that is connected to the operating rod (32) at its distal end that faces away from the mixing element (34), and wherein the hand grip (33) is designed, by way of the second hand of the user, to manually move the operating rod (32) to and fro along the longitudinal direction and to rotate it while the user holds the housing (10) with the first hand.

7. The mixing and discharge system according to claim 6, wherein the hand grip (33) is designed so as to open out in a wedge-shaped manner in the distal direction and is asymmetrical relative to a rotation on the longitudinal direction.

8. The mixing and discharge system according to any one of the preceding claims, wherein the operating rod (32) is non-detachably connected to the mixing element (34).

9. The mixing and discharge system according to any one of the preceding claims, wherein an external thread (13) is formed on the housing (10) in the region of the distal end (15) of the mixing chamber (14), and wherein the first closure cap (31) comprises a jacket region with an internal thread (37) that engages the external thread (13).

10. The mixing and discharge system according to any one of the preceding claims, wherein the open distal end (15) of the mixing chamber (14) forms an access opening to the mixing chamber (14), with the diameter of said access opening being at least as large as the diameter of the mixing chamber (14) in a cylindrical region that is situated so as to be proximally spaced apart from the distal end (15), in which cylindrical region the piston (23) is displaceable.

11. The mixing and discharge system according to any one of the preceding claims, wherein the second closure cap (40) comprises a connecting region (43) for an accessory part, which connecting region (43) encompasses the discharge opening (44), and comprises a jacket surface that conically tapers in the distal direction and that is preferably designed as a male Luer cone.

12. The mixing and discharge system according to claim 11, wherein the second closure cap (40) comprises a securing sleeve (42) which radially encompasses the connecting region (43) and which is arranged so as to be non-rotational or rotational, with an internal thread facing the connecting region (43), in order to secure the accessory part on the connecting region.

13. The mixing and discharge system according to claim 11 or 12 which, furthermore, comprises a closure part (50) that can be detachably attached to the connecting region (43) in order to close the discharge opening (44).

14. The mixing and discharge system according to any one of the preceding claims, further comprising at least one accessory part that is adapted to be detachably connected to the discharge opening (44) or is non-detachably connected to the discharge opening (44) and that is selected from the following group of accessory parts:
a discharge nozzle;
a spraying device;
a hollow needle (60);
a mixing tube (70; 80).

## Revendications

1. Système de mélange et de déversement qui présente un dispositif de mélange pour mélanger un produit à mélanger, dans lequel le dispositif de mélange présente :
un boîtier (10) qui délimite une chambre de mélange (14) essentiellement cylindrique, sachant que la chambre de mélange (14) définit un sens longitudinal et présente une extrémité distale ouverte (15) et une extrémité proximale ouverte, et sachant que le boîtier est ainsi dimensionné qu'il peut être saisi entre deux doigts d'une première main d'un utilisateur ;
un piston (23) disposé dans la chambre de mélange (14) de façon à pouvoir être déplacé dans le sens longitudinal, lequel délimite la chambre de mélange en étanchéité en direction de l'extrémité proximale ;
une tige de piston (21) reliée au piston (23) qui fait saillie de l'extrémité proximale de la chambre de mélange (14) et qui présente un repose-pouce (22), sachant que le repose-pouce (22) est ainsi disposé qu'il permet à l'utilisateur de faire avancer la tige de piston (21) dans le sens distal avec le pouce de la première main lorsque l'utilisateur tient le boîtier (10) entre deux doigts de la première main ;
un élément mélangeur (34) pouvant être déplacé le long du sens longitudinal dans la chambre de mélange (14) ;
une tige d'actionnement (32) reliée de façon distale à l'élément mélangeur (34), laquelle fait saillie de l'extrémité distale ouverte de la chambre de mélange (14) ; et
un premier bouchon de fermeture (31) qui est en prise de façon séparable avec le boîtier (10) sur l'extrémité distale ouverte (15) de la chambre de mélange (14), qui ferme l'extrémité distale ouverte (15) de la chambre de mélange (14) et qui présente une ouverture de passage à travers laquelle la tige d'actionnement (32) est dirigée, sachant que le premier bouchon de fermeture (31) peut être enlevé complètement du boîtier (10) conjointement avec la tige d'actionnement (32) et l'élément mélangeur (34) ;
**caractérisé en ce que** le dispositif de mélange et de déversement comprend en outre un second bouchon de fermeture (40) qui, après le retrait du premier bouchon de fermeture (31), peut être mis en prise de façon séparable avec le boîtier (10) au niveau de l'extrémité distale ouverte (15) de la chambre de mélange (14) pour fermer l'extrémité distale ouverte (15) de la chambre de mélange (14), et qui présente une ouverture de déversement (44) à travers laquelle un produit de mélange reçu dans la chambre de mélange (14) peut être déversé dans le sens distal via une pression sur la tige de piston (21).

2. Système de mélange et de déversement selon la revendication 1, dans lequel le premier bouchon de fermeture (31) présente un élément de guidage (35) qui s'étend à l'intérieur de la chambre de mélange (14), qui repose sur au moins trois points, de préférence périphériques, sur une paroi de délimitation (11) à l'intérieur de la chambre de mélange (14) et dans lequel la tige d'actionnement (32) est dirigée.

3. Système de mélange et de déversement selon la revendication 1 ou 2, dans lequel un creux (36) se rétrécissant dans le sens distal est formé dans une partie d'extrémité proximale du premier bouchon de fermeture (31), afin de recevoir une extrémité distale de l'élément mélangeur (34) lorsque l'élément mélangeur (34) se trouve dans une position distale finale.

4. Système de mélange et de déversement selon l'une des revendications précédentes, dans lequel le piston (23) présente une partie d'extrémité se rétrécissant dans le sens distal et dans lequel l'élément mélangeur (34) forme une réception se rétrécissant dans le sens distal pour la partie d'extrémité du piston (23).

5. Système de mélange et de déversement selon l'une des revendications précédentes, dans lequel le premier bouchon de fermeture présente une lèvre d'enlèvement périphérique qui repose sur la tige d'actionnement.

6. Système de mélange et de déversement selon l'une des revendications précédentes, dans lequel le dispositif mélangeur présente une poignée (33) qui est reliée à la tige d'actionnement (32) par son extrémité distale détournée de l'élément mélangeur (34) et dans lequel la poignée (33) est ainsi formée pour déplacer et tourner la tige d'actionnement (32) manuellement d'avant en arrière avec une seconde main de l'utilisateur dans le sens longitudinal pendant que l'utilisateur tient le boîtier (10) avec la première main.

7. Système de mélange et de déversement selon la revendication 6, dans lequel la poignée (33) est formée en s'élargissant en forme de coin et en asymétrie dans le sens distal par rapport à une rotation sur le sens longitudinal.

8. Système de mélange et de déversement selon l'une des revendications précédentes, dans lequel la tige d'actionnement (32) est reliée fixement à l'élément mélangeur (34).

9. Système de mélange et de déversement selon l'une des revendications précédentes, dans lequel un filetage mâle (13) est formé au niveau de l'extrémité distale (15) de la chambre de mélange (14) sur le boîtier (10), et sachant que le premier bouchon de fermeture (31) présente une partie d'enveloppe avec un filetage femelle (37) qui est en prise avec le filetage mâle (13).

10. Système de mélange et de déversement selon l'une des revendications précédentes, dans lequel l'extrémité distale ouverte (15) de la chambre de mélange (14) forme une ouverture d'accès vers la chambre de mélange (14) dont le diamètre est au moins égal au diamètre de la chambre de mélange (14) dans une partie cylindrique placée à distance proximale de l'extrémité distale (15) dans laquelle le piston (23) peut se déplacer.

11. Système de mélange et de déversement selon l'une des revendications précédentes, dans lequel le second bouchon de mélange (40) présente une partie de connexion (43) pour un accessoire qui entoure l'ouverture de déversement (44) et qui présente une surface d'enveloppe conique dans le sens distal, et qui est conçue de préférence en tant que cône luer mâle.

12. Système de mélange et de déversement selon la revendication 11, dans lequel le second bouchon de mélange (40) présente un manchon de blocage (42) fixe en rotation ou rotatif, entourant la partie de connexion (43) dans le sens radial, avec un filetage femelle orienté vers la partie de connexion (43) pour bloquer l'accessoire sur la partie de connexion.

13. Système de mélange et de déversement selon la revendication 11 ou 12, lequel présente en outre une partie de fermeture (50) pouvant être fixée de façon séparable sur la partie de connexion (43) afin de fermer l'ouverture de déversement (44).

14. Système de mélange et de déversement selon l'une des revendications précédentes, lequel présente en outre au moins un accessoire qui peut être relié de façon séparable à l'ouverture de déversement (44) ou est relié fixement à l'ouverture de déversement (44) et qui est sélectionné parmi le groupe suivant d'accessoires
une buse de déversement ;
un dispositif de pulvérisation ;
une aiguille creuse (60) ;
un tube mélangeur (70 ; 80) :
